# EUROPEAN PATENT APPLICATION

(11) **EP 4 585 607 A1**
(43) Date of publication of application: **16.07.2025**
(21) Application number: 24756343.0
(22) Date of filing: 15.02.2024
(51) Int. Cl.: C07K 5/078, C07K 1/08

(54) **INTERMEDIATE USED FOR FAPI SYNTHESIS, PREPARATION METHOD THEREFOR, AND APPLICATION THEREOF**

(30) Priority: 16.02.2023 CN 202310124318
(71) Applicant: Yantai Lannacheng Biotechnology Co., Ltd., Yantai Shandong 264199 (CN)
(72) Inventor: WU, Xiaoming, Yantai, Shandong 264199 (CN); WANG, Xiangyu, Yantai, Shandong 264199 (CN); HE, Tian, Yantai, Shandong 264199 (CN); YANG, Qingbao, Yantai, Shandong 264199 (CN); CHENG, Lin, Yantai, Shandong 264199 (CN)
(74) Representative: Wang, Bo
(86) International application number: PCT/CN2024/077215
(87) International publication number: WO 2024/169973

(57) **Abstract**

The present invention provides an intermediate used for Evans blue modified FAPI synthesis, a method for preparing the intermediate, and an application of the intermediate in the synthesis of an Evans blue modified FAPI. The use of the intermediate provided by the present invention to synthesize Evans blue modified FAPI can improve the production efficiency and reduce the production cost, and the intermediate is suitable for industrial production.

## Description

### TECHNICAL FIELD

The present invention relates to the field of synthesis of drugs, and specifically relates to an intermediate for synthesis of Evans blue modified FAPI (LNC1004), a preparation method therefor and use thereof.

### BACKGROUND

Molecular imaging radioactive tracers targeting a fibroblast activation protein (FAP) have shown good preclinical and clinical results in tumor diagnosis.LNC1004, as a newly developed Evans blue modified fibroblast activation protein inhibitor (Evans blue modified FAPI), has a chemical name of trifluoroacetate 2,2',2''-(10-(2-(((S)-1-((4'-((E)-(8-amino-1-hydroxy-5,7-disulfonaphthalen-2)-yl)diazenyl)-3, 3'-dimethyl-[1,1'-biphenyl]-4-yl)amino)-6-(4-(4-(3-((4-((2-((S)-2-cyanopyrrolidin-1-yl)-2-ox oethyl)carbamoyl)quinolin-6-yl)oxy)propyl)(piperazin-1-yl)-4-oxobutanamid)-1-oxohexyl-2-y l)amino)-2-oxoethyl)-1,4,7,10-tetraazacyclododecan-1,4,7-triyl)triacetate. In the prior art, the LNC1004 is used for targeted therapy of tumors after being labeled with ¹⁷⁷Lu. It has been found that the addition of an Evans blue fragment can prolong the cycle half-life to improve pharmacokinetics (PK), thereby increasing tumor uptake and improving a radiotherapy effect. Therefore, the radiolabeled LNC1004 can be used as a novel long-acting cancer therapeutic drug.

A synthesis route of the LNC1004 has been reported in the prior art (document: Evans blue-modified radiolabeled fibroblast activation protein inhibitor as long-acting cancer therapeutics, Theranostics 2022; 12(1): 422-433):

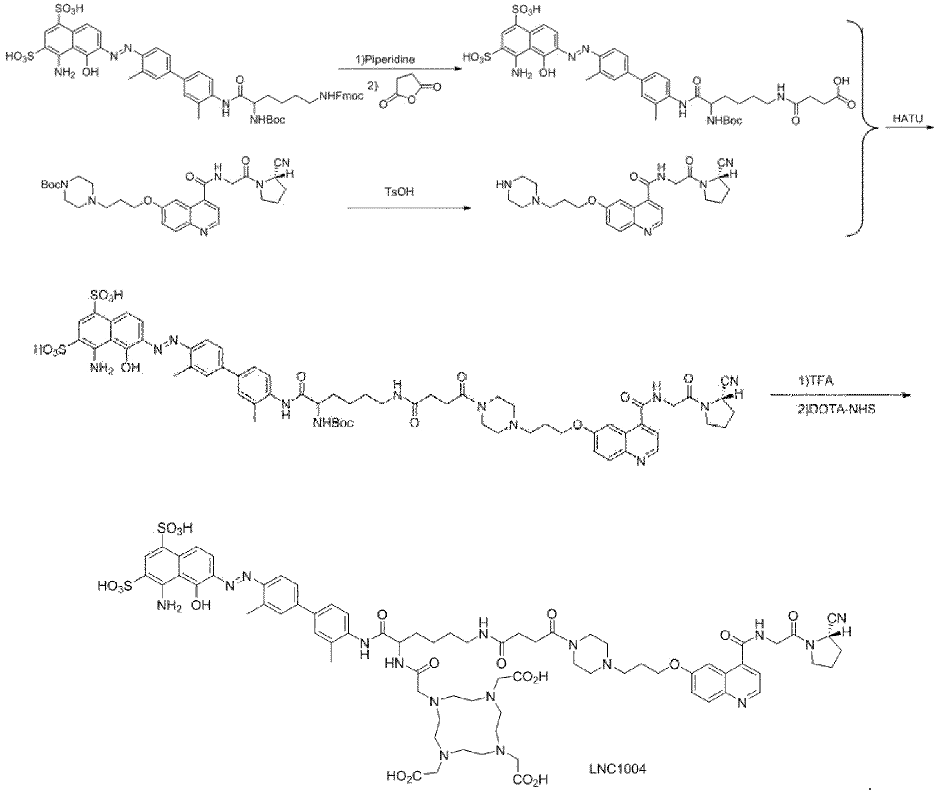

Although the target product can be obtained, the above route has the following disadvantages: (1) an Evans blue fragment with a sulfonic acid group is used as a starting material to synthesize the Evans blue modified FAPI (LNC1004), such that the polarity of an intermediate involved in the whole synthesis route is increased, separation and purification are difficult to perform by using traditional post-treatment means (such as extraction, column chromatography, etc.), meanwhile the use of purification by preparative liquid chromatography increases the separation difficulty and has low efficiency, and the route is not suitable for industrial production; and (2) DOTA-NHS is used for coupling of final fragments, and the DOTA-NHS itself is unstable in structure and pone to decomposition and ring opening at a high temperature, resulting in more side reactions and a low yield; and moreover, the DOTA-NHS itself is expensive, resulting in a high overall cost.

Therefore, in order to facilitate industrial enlarged production of the LNC1004, it is necessary to improve synthesis methods thereof.

### SUMMARY

In view of the above technical background, a technical problem to be solved by the present invention is how to increase the synthesis yield of LNC1004 and reduce the cost to make the LNC1004 more suitable for industrial enlarged production.

A primary purpose of the present invention is to provide a novel compound as a key intermediate for synthesis of Evans blue modified FAPI. By using the key intermediate to synthesize the Evans blue modified FAPI, not only can the production cost be reduced, but also the production efficiency and the yield can be significantly improved, and the key intermediate is suitable for large-scale industrial production.

A second purpose of the present invention is to provide a method for preparing the key intermediate.

Another purpose of the present invention is to provide a method for synthesizing Evans blue modified FAPI by using the key intermediate, that is, use of the key intermediate in synthesis of Evans blue modified FAPI.

In order to achieve the above purposes, the following technical solutions are adopted in the present invention.

In a first aspect, the present invention provides an intermediate for synthesis of Evans blue modified FAPI (LNC1004), which has a structure as shown in Formula (I):

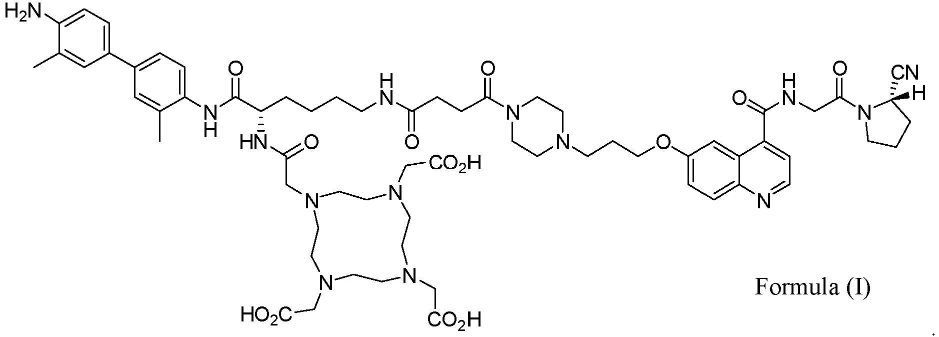

In a second aspect, the present invention provides a method for preparing the intermediate (that is, the compound of Formula I), including the following steps:
step a: subjecting (S)-N-(2-(2-cyanopyrrolidin-1-yl)-2-oxoethyl)-6-(3-(piperazin-1-yl) propoxy)quinolin-4-formamide (that is, a compound II) to a reaction with succinic anhydride to obtain a compound III;
step b: subjecting the compound III to a coupling reaction with tert-butyl (S)-(6-amino-1-((4'-amino-3,3'-dimethyl-[1,1'-biphenyl]-4-yl)amino)-1-oxohexyl-2-yl)carba mate (that is, a compound IV) and treatment to obtain a compound V;
step c: subjecting the compound V to a deprotection reaction to obtain a compound VI, and then to a substitution reaction and treatment to obtain a compound VII; and
step d: subjecting the compound VII to a hydrolysis reaction and treatment to obtain the intermediate having the structure of Formula I.

Specifically, a reaction route of the intermediate (that is, the compound of Formula I) of the present invention is as follows:

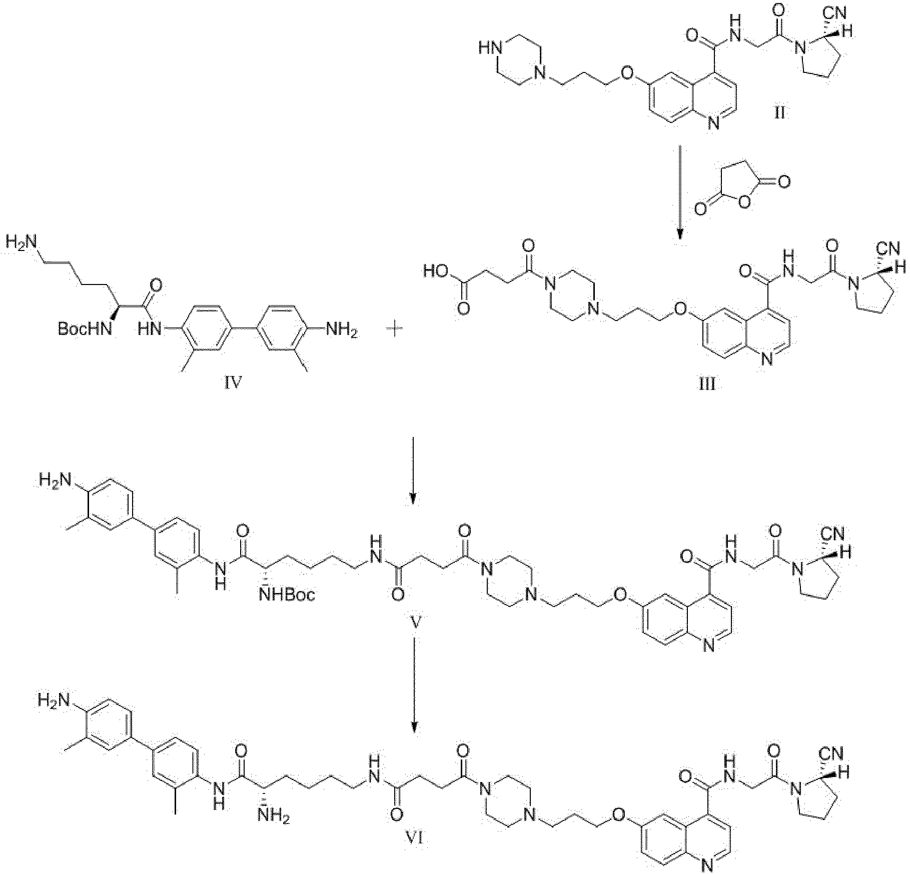

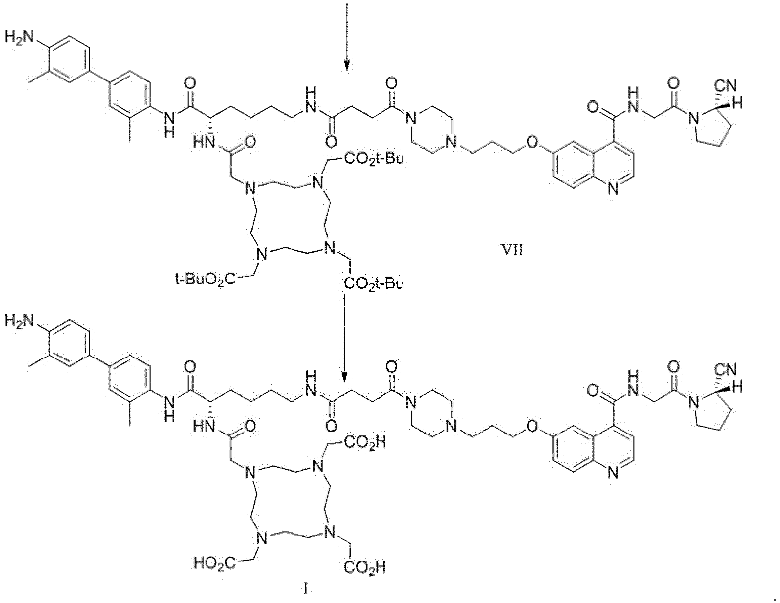

In a preferred solution of the present invention, the step b specifically includes: in an N,N-dimethylformamide solvent, adding the compound III obtained in the step a and the tert-butyl (S)-(6-amino-1-((4'-amino-3,3'-dimethyl-[1,1'-biphenyl]-4-yl)amino)-1- oxohexyl-2-yl)carbamate (that is, the compound IV), then adding a condensing agent and an organic alkali, performing stirring for a reaction at 20-45°C for 1-8 hours, and performing treatment to obtain the compound V.

In a more preferred solution of the present invention, the condensing agent in the step b includes any one of HATU, HBTU, TBTU, TSTU, PyAOP, and PyBOP, most preferably HATU; and the organic alkali in the step b includes any one of N,N-diisopropylethylamine and triethylamine, most preferably N,N-diisopropylethylamine.

In a preferred solution of the present invention, the step c specifically includes: in an organic solvent, adding the compound V obtained in the step b, then adding an organic acid, performing stirring for a reaction at 20-45°C for 1-8 hours to obtain the compound VI, then adding an excess amount of an organic alkali, adding tri-tert-butyl 2,2',2''-(10-(2-((2,5-dioxopyrrolidin-1-yl)oxy)-2-oxoethyl)-1,4,7,10-tetraazacyclododecan-1,4,7-triyl)triacetate (DOTA-TRIS-TBU-ESTER NHS), performing stirring for a reaction at 15-45°C for 1-8 hours, and performing treatment to obtain the compound VII.

In a more preferred solution of the present invention, the organic solvent in the step c includes any one of dichloromethane, N,N-dimethylformamide, or a mixture of the two, most preferably N,N-dimethylformamide; the organic acid in the step c includes any one of trifluoroacetic acid and p-toluenesulfonic acid, most preferably trifluoroacetic acid; and the organic alkali in the step c includes any one of N,N-diisopropylethylamine and triethylamine, most preferably N,N-diisopropylethylamine.

In a preferred solution of the present invention, the step d specifically includes: in an organic solvent, adding the compound VII obtained in the step c, then adding an organic acid, performing stirring for a reaction at 20-45°C for 1-8 hours, and performing treatment to obtain the intermediate having the structure of Formula I.

In a more preferred solution of the present invention, the organic solvent in the step d is dichloromethane or acetonitrile.

In a more preferred solution of the present invention, the organic acid in the step d includes any one of trifluoroacetic acid and p-toluenesulfonic acid, most preferably trifluoroacetic acid.

The present invention provides a method for preparing the intermediate (that is, the compound of Formula I), the compound of Formula (I) is prepared by a hydrolysis reaction of a compound of Formula (VII), and a preparation route is as follows:

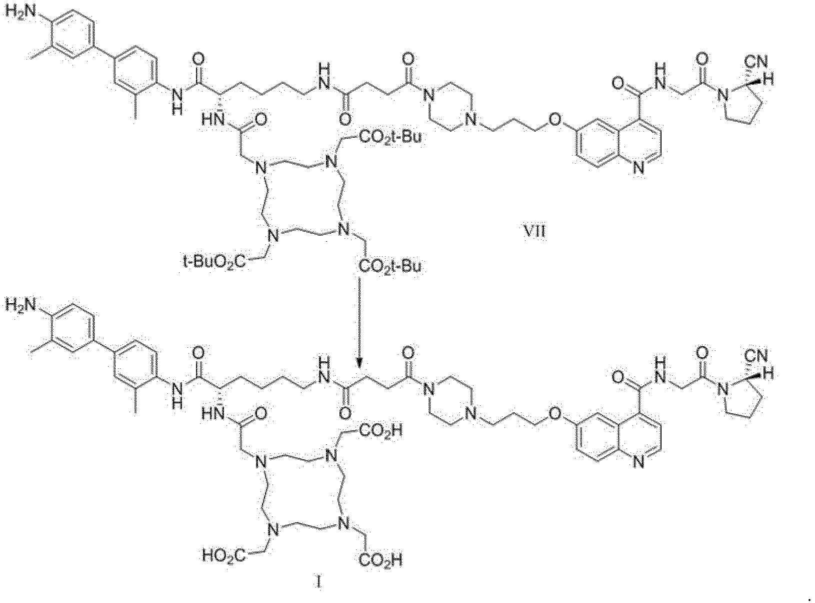

A preferred preparation method for the compound of Formula (I) using the compound of Formula (VII) includes: in an organic solvent, adding the compound of Formula (VII), then adding an organic acid, performing stirring for a reaction at 20-45°C for 1-8 hours, and performing treatment to obtain the intermediate having the structure of Formula I.

Preferably, the organic solvent in the above preparation method is preferably dichloromethane or acetonitrile.

Preferably, the organic acid in the above preparation method preferably includes any one of trifluoroacetic acid and p-toluenesulfonic acid, most preferably trifluoroacetic acid.

Further, the compound of Formula (VII) is obtained by a substitution reaction of a compound of Formula (VI), and a preparation route is as follows:

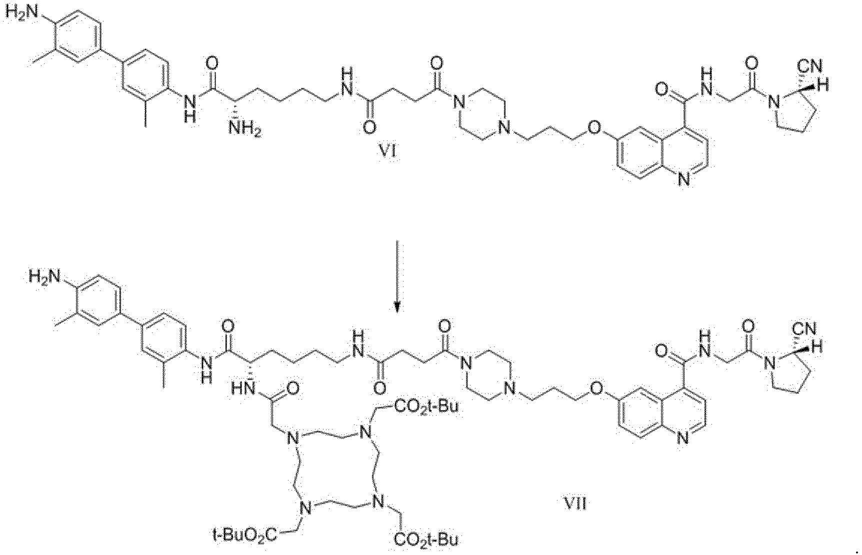

A preferred preparation method for the compound of Formula (VII) using the compound of Formula (VI) includes: in an organic solvent, adding the compound VI, then an excess amount of an organic alkali, adding tri-tert-butyl 2,2',2''-(10-(2-((2,5-dioxopyrrolidin-1-yl) oxy)-2-oxoethyl)-1,4,7,10-tetraazacyclododecan-1,4,7-triyl)triacetate (DOTA-TRIS-TBU-ESTER NHS), performing stirring for a reaction at 15-45°C for 1-8 hours, and performing treatment to obtain the compound VII.

Preferably, the organic solvent in the above preparation method includes any one of dichloromethane, N,N-dimethylformamide, or a mixture of the two, most preferably N,N-dimethylformamide.

Preferably, the organic alkali in the above preparation method includes any one of N,N-diisopropylethylamine and triethylamine, most preferably N,N-diisopropylethylamine.

Furthermore, the compound of Formula (VI) is obtained by deprotection of a compound of Formula (V), and a preparation route is as follows:

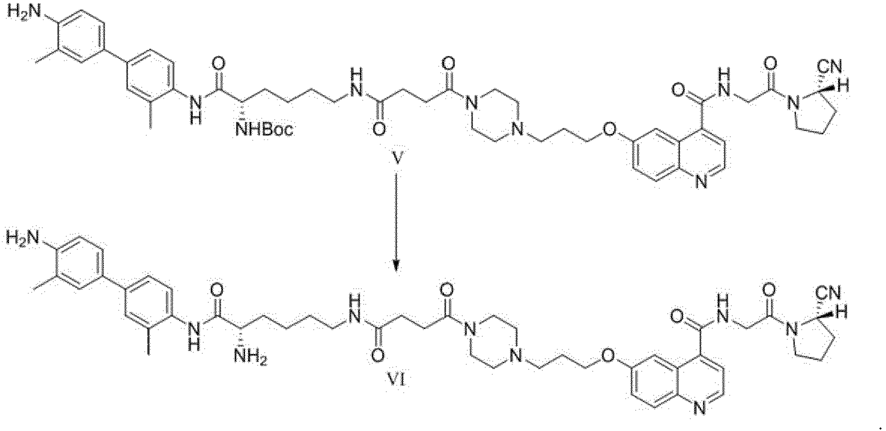

A preferred preparation method for the compound of Formula (VI) using the compound of Formula (V) includes: in an organic solvent, adding the compound V, then adding an organic acid, and performing stirring for a reaction at 20-45°C for 1-8 hours to obtain the compound VI.

Preferably, the organic solvent in the above preparation method includes any one of dichloromethane, N,N-dimethylformamide, or a mixture of the two, most preferably N,N-dimethylformamide.

Preferably, the organic acid in the above preparation method includes any one of trifluoroacetic acid and p-toluenesulfonic acid, most preferably trifluoroacetic acid.

Furthermore, the compound of Formula (V) is obtained by coupling of a compound of Formula (IV) and a compound of Formula (III), and a preparation route is as follows:

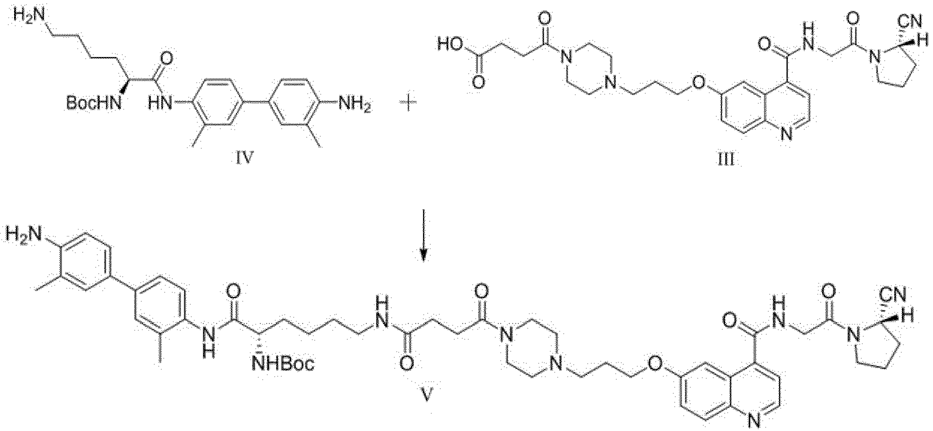

A preferred preparation method for the compound of Formula (V) using the compound of Formula (IV) and the compound of Formula (III) includes: in an N,N-dimethylformamide solvent, adding the compound III and the compound IV, then adding a condensing agent and an organic alkali, performing stirring for a reaction at 20-45°C for 1-8 hours, and performing treatment to obtain the compound V.

Preferably, the condensing agent in the above preparation method includes any one of HATU, HBTU, TBTU, TSTU, PyAOP, and PyBOP, most preferably HATU.

Preferably, the organic alkali in the above preparation method includes any one of N,N-diisopropylethylamine and triethylamine, most preferably N,N-diisopropylethylamine.

Furthermore, the compound of Formula (III) is obtained by a reaction of a compound of Formula (II) and succinic anhydride, and a preparation route is as follows:

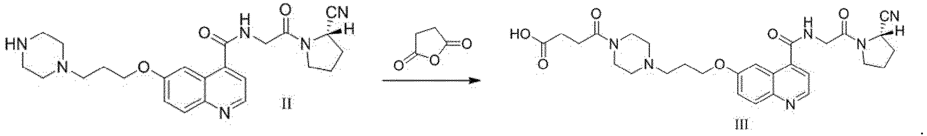

In a third aspect, the present invention provides a method for synthesizing Evans blue modified FAPI (LNC1004) by using the intermediate (that is, the compound of Formula I) of the present invention, and the present invention further provides use of the intermediate (that is, the compound of Formula I) in synthesis of Evans blue modified FAPI (LNC1004).

Specifically, the above method or use includes the following reaction route:

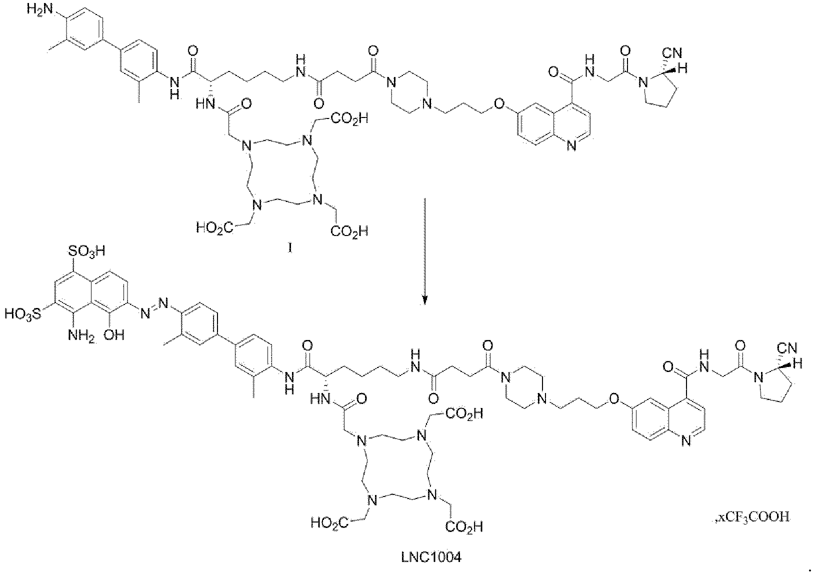

The above method or use includes reaction steps: dissolving the intermediate having the structure of Formula (I) in water, sequentially adding a hydrochloric acid solution and sodium nitrite for a reaction to generate a diazonium salt solution under stirring conditions at -5°C to 25°C, then mixing a 1-amino-8-naphthol-2,4-disulfonic acid monosodium salt and sodium bicarbonate for an acid-base neutralization reaction to generate an aqueous solution of the 1-amino-8-naphthol-2,4-disulfonic acid monosodium salt and the sodium bicarbonate, dropping the diazonium salt solution into the aqueous solution of the 1-amino-8-naphthol-2,4-disulfonic acid monosodium salt and the sodium bicarbonate for a reaction for 1-8 hours under stirring at -5°C to 25°C, and performing post-treatment to obtain Evans blue modified FAPI (that is, LNC1004).

In a preferred solution of the present invention, in the reaction steps, a molar concentration of the hydrochloric acid solution is 0.5-6 mol/L, more preferably 1-3 mol/L; a molar ratio of an equivalent of hydrochloric acid to that of the intermediate is 1.0-3.0, more preferably 1.5-2.0; a molar ratio of an equivalent of the sodium nitrite to that of the intermediate is 1.0-3.0, more preferably 1.0-1.5; a molar ratio of an equivalent of the 1-amino-8-naphthol-2,4-disulfonic acid monosodium salt to that of the intermediate is 1.0-3.0, more preferably 1.0-1.5; and a molar ratio of an equivalent of the sodium bicarbonate to that of the intermediate is 5.0-10.0, more preferably 8.0-10.0.

The method for synthesizing Evans blue modified FAPI (LNC1004) of the present invention is used on the premise of synthesizing the intermediate (the compound of Formula I) of the present invention, and finally the LNC1004 is obtained by coupling of the intermediate and the 1-amino-8-naphthol-2,4-disulfonic acid monosodium salt. Compared with existing synthesis methods, the integral synthesis method of the present invention mainly has the differences that the reaction with the 1-amino-8-naphthol-2,4-disulfonic acid monosodium salt is placed in the last step; and a way of first coupling with a carboxyl-protected DOTA group and then deprotection is used during introduction of a DOTA group. Based on the above differences, the present invention has the following advantages.
(1) By placing the coupling of the 1-amino-8-naphthol-2,4-disulfonic acid monosodium salt and the intermediate in the last step of synthesis, the polarity of the reaction intermediate is reduced compared with existing synthesis methods, such that compounds prepared before obtaining the intermediate in the synthesis route can all be separated and purified by extraction, column chromatography and other post-treatment means. Thus, the production efficiency and yield of a whole synthesis process are obviously improved, and the method is suitable for large-scale industrial production.
(2) By using the way of first coupling with a carboxyl-protected DOTA group and then deprotection during introduction of a DOTA group, that is, using the tri-tert-butyl 2,2',2''-(10-(2-((2,5-dioxopyrrolidin-1-yl)oxy)-2-oxoethyl)-1,4,7,10-tetraazacyclododecan-1, 4,7-triyl)triacetate (DOTA-TRIS-TBU-ESTER NHS) to replace DOTA-NHS to participate in a reaction and subsequently removing a tert-butyl ester, the synthesis process can be more efficient, the yield is higher, and the cost is lower. First, since the tri-tert-butyl 2,2',2''-(10-(2-((2,5-dioxopyrrolidin-1-yl)oxy)-2-oxoethyl)-1,4,7,10-tetraazacyclododecan-1,4,7-triyl)triacetate (DOTA-TRIS-TBU-ESTER NHS) has more stable chemical properties than the DOTA-NHS and has a structure difficult to destroy in the reaction, the present invention has few side reactions and a higher single-step reaction yield. Second, since the tri-tert-butyl 2,2',2''-(10-(2-((2,5-dioxopyrrolidin-1 -yl)oxy)-2-oxoethyl)-1,4,7,10-tetraazacyclododecan-1,4,7-triyl)triacetate (DOTA-TRIS-TBU-ESTER NHS) has lower polarity than the DOTA-NHS, the corresponding compound (VII) can still be separated and purified by column chromatography after being obtained in the reaction, and the method is suitable for industrial production. Third, since the tri-tert-butyl 2,2',2''-(10-(2-((2,5 -dioxo-pyrrolidin-1-yl)oxy)-2-oxoethyl)-1,4,7,10-tetraazacyclododecan- 1,4,7-triyl)triacetate (DOTA-TRIS-TBU-ESTER NHS) has a lower market price (about 1,500 yuan/g) than the DOTA-NHS (about 10,000 yuan/g), the tri-tert-butyl 2,2',2''-(10-(2-((2,5-dioxo-pyrrolidin -1-yl)oxy)-2-oxoethyl)-1,4,7,10-tetraazacyclododecan-1,4,7-triyl)triacetate (DOTA-TRIS-TBU-ESTER NHS) is selected in the present invention to replace the DOTA-NHS, which can greatly reduce the production cost.

### BRIEF DESCRIPTION OF DRAWINGS

FIG. 1 is a mass spectrogram of a compound III in Example 1.
FIG. 2 is a mass spectrogram of a compound V in Example 1.
FIG. 3 is a mass spectrogram of a compound VII in Example 1.
FIG. 4 is a mass spectrogram of an intermediate of Formula I in Example 1.
FIG. 5 is a mass spectrogram of Evans blue modified FAPI (LNC1004) in Example 2.

### DETAILED DESCRIPTION OF EMBODIMENTS

The present invention may be better understood according to the following examples. However, those skilled in the art easily understand that specific material ratios, process conditions and results described in the examples are intended only to illustrate the present invention, and shall not and will not limit the present invention described in detail in the claims.

### Example 1: Preparation of a compound of Formula (I)

### Preparation of a compound III

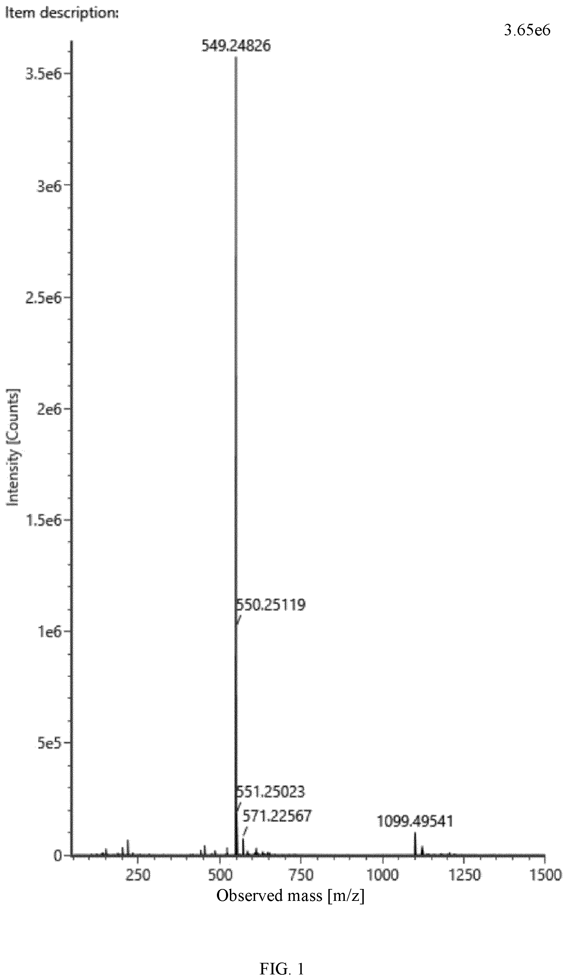

10.06 g of (S)-N-(2-(2-cyanopyrrolidin-1-yl)-2-oxoethyl)-6-(3-(piperazin-1-yl) propoxy)quinolin-4-formamide (that is, a compound II) was added to 70 ml of dichloromethane for dissolution, then 16.9 ml of DIPEA and 2.81 g of succinic anhydride were sequentially added, stirring was performed for a reaction at room temperature for 2 hours, and purification was performed by column chromatography to obtain 7.32 g of a compound III with a yield of 59.54%. A theoretical [M-H]⁻ value is 549.25, and a measured [M-H]⁻ value is 549.24826.A structural characterization spectrogram is shown in FIG. 1.

### Preparation of a compound V

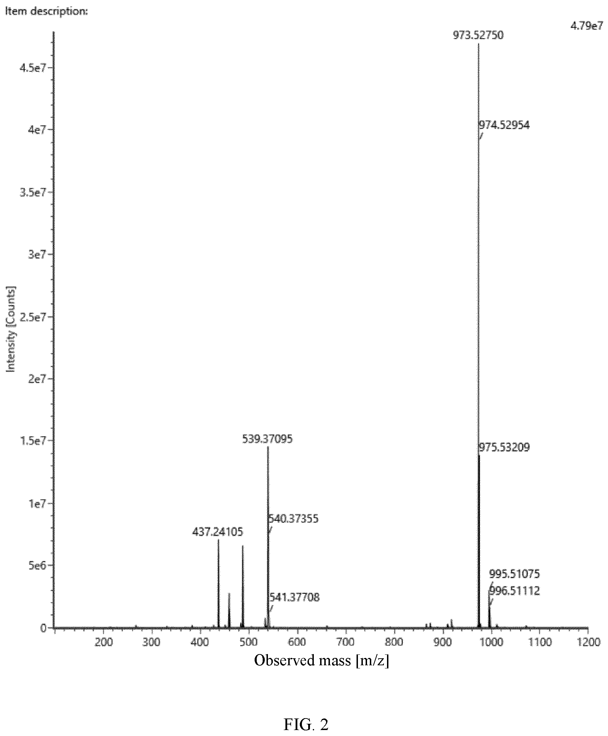

In 70 ml of N,N-dimethylformamide, 7.20 g of the compound III, 5.83 g of (S)-(6-amino-1-((4'-amino-3,3'-dimethyl -[1,1'-biphenyl]-4-yl)amino)-1-oxohexyl-2-yl) carbamate (a compound IV), 6.12 g of HATU, and 2.66 g of DIPEA were sequentially added and stirring for a reaction at room temperature for 2 hours, and purification was performed by column chromatography to obtain 11.66 g of a compound V with a yield of 91.30%. A theoretical [M+H]⁺ value is 973.53, and a measured [M+H]⁺ value is 973.52750.A structural characterization spectrogram is shown in FIG. 2.

### Preparation of a compound VII

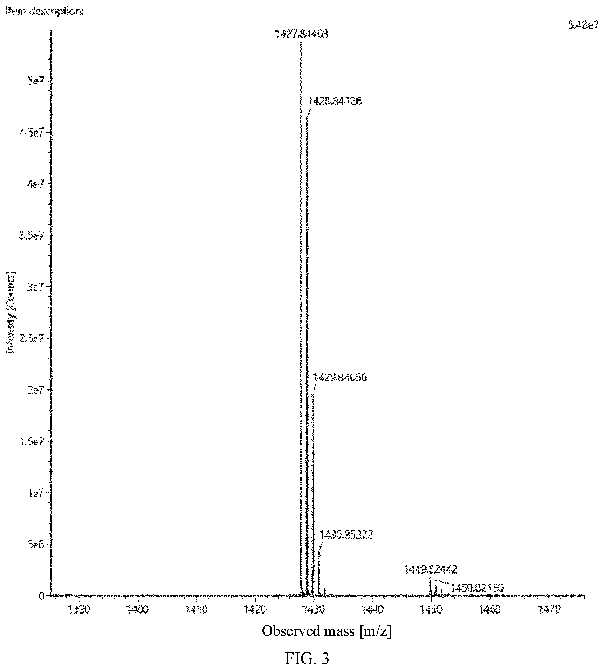

In 200 ml of N,N-dimethylformamide, 11.50 g of the compound V was added, then 77.86 ml of trifluoroacetic acid was added, and stirring was performed for a reaction at 40-45°C for 1 hour to obtain a compound VI. Then, 234 ml of N,N-diisopropylethylamine was added, 7.5 g of tri-tert-butyl 2,2',2''-(10-(2-((2,5-dioxo -pyrrolidin-1-yl)oxy)-2-oxoethyl)-1,4,7,10-tetraazacyclododecan-1,4,7-triyl)triacetate (DOTA-TRIS-TBU-ESTER NHS) was added, and stirring was performed for a reaction at room temperature for 6 hours. A reaction solution was spin-dried and dissolved in 200 ml of dichloromethane, and an organic phase was washed with water for 2 times, dried with anhydrous sodium sulfate, then spin-dried, and purified by column chromatography to obtain 20.85 g of a compound VII with a yield of 123.51% (containing an inorganic salt). A theoretical [M+H]⁺ value is 1427.85, and a measured [M+H]⁺ value is 1427.84403.A structural characterization spectrogram is shown in FIG. 3.

### Preparation of a compound of Formula (I)

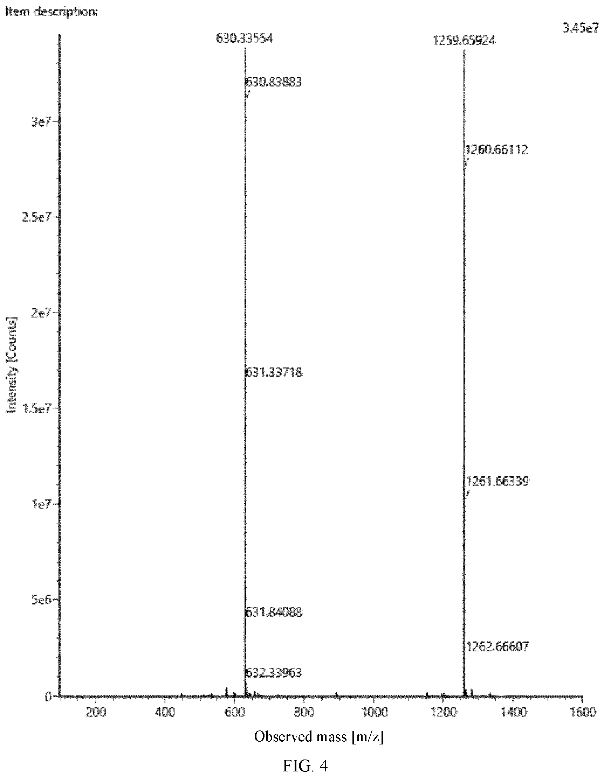

20.5 g of the compound VII was taken, 350 ml of dichloromethane and 350 ml of trifluoroacetic acid were added, and stirring was performed for a reaction at 40-45°C for 1 hour. A reaction solution was dropped into 3.5 L of methyl tert-butyl ether, stirred to precipitate a solid, filtered, and purified by preparative liquid chromatography to obtain 10.36 g of a compound of Formula I with a yield of 56.30%. A theoretical [M+H]⁺ value is 1259.66, a theoretical ([M+2H]/2)⁺ value is 630.33, and a measured ([M+2H]/2)⁺ value is 630.33554.A structural characterization spectrogram is shown in FIG. 4.

A reaction route in the present example is as follows:

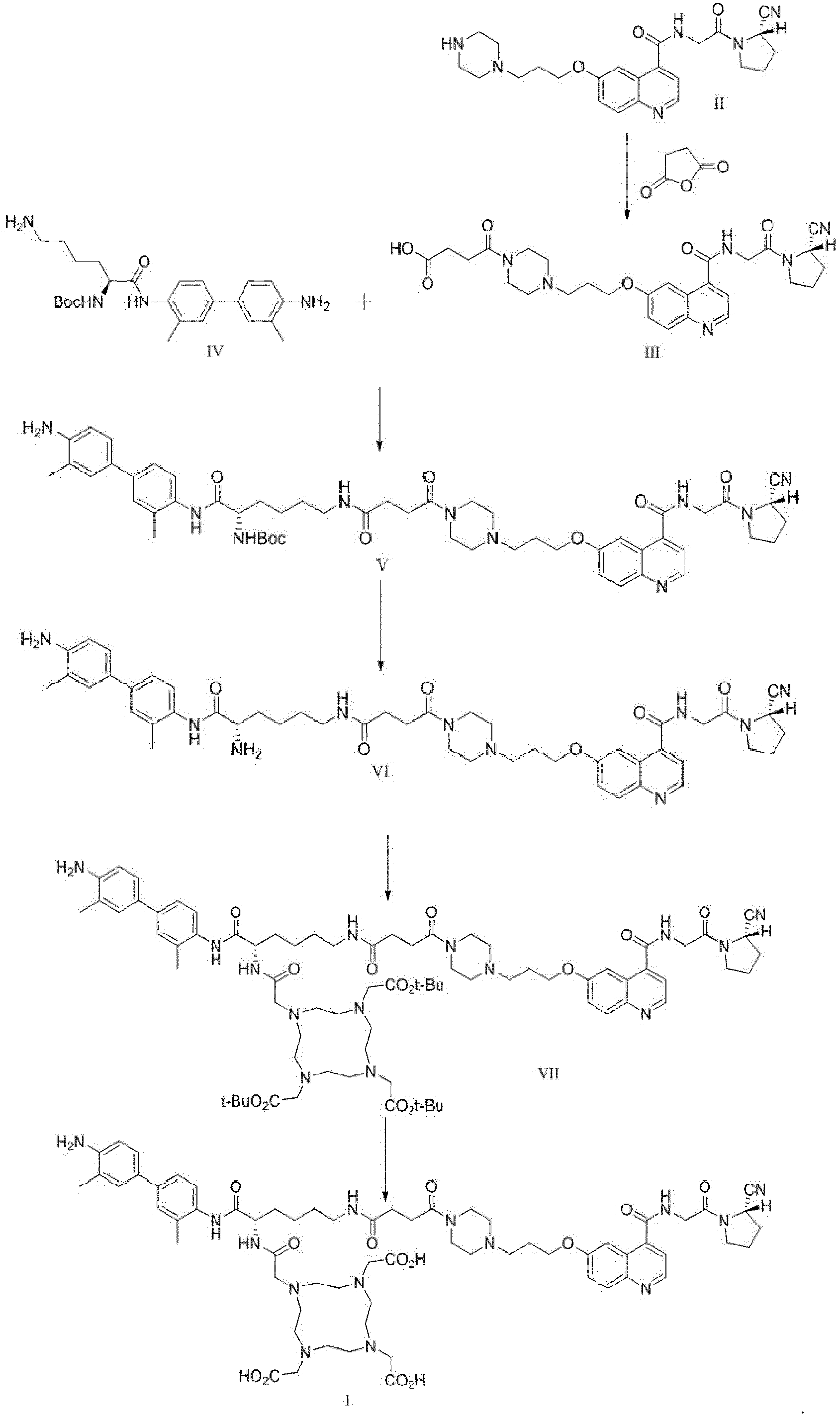

### Example 2: Preparation of Evans blue modified FAPI (LNC1004)

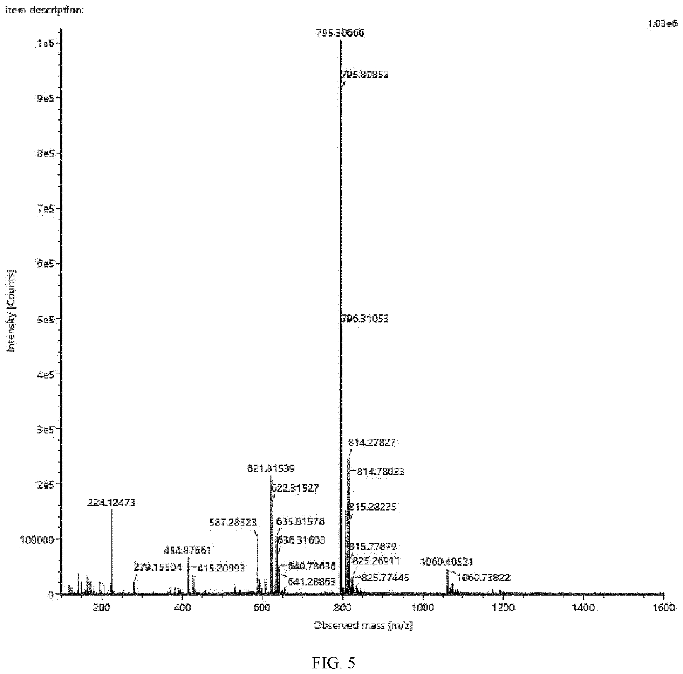

10.3 g of the compound of Formula (I) prepared in Example 1 was dissolved in 135 ml of water, and 8.7 ml of a 2 mol/L hydrochloric acid solution and 0.618 g of sodium nitrite were sequentially added for a reaction to generate a diazonium salt solution under stirring at 0-10°C. Then, 6.9 g of sodium bicarbonate was dissolved in 135 ml of water, and 3.1 g of a 1-amino-8-naphthol-2,4-disulfonic acid monosodium salt was added for an acid-base neutralization reaction to generate an aqueous solution of the 1-amino-8-naphthol-2,4-disulfonic acid monosodium salt and the sodium bicarbonate. Under stirring at 0-10°C, the diazonium salt solution was dropped into the aqueous solution of the 1-amino-8-naphthol-2,4-disulfonic acid monosodium salt and the sodium bicarbonate for a reaction for 1 hour. A reaction solution was spin-dried and purified by preparative liquid chromatography to obtain 7.01 g of an Evans blue modified fibroblast activation protein inhibitor (LNC1004) capable of being radiolabeled with a yield of 53.91%. A theoretical ([M+2H]/2)⁺ value is 795.31, and a measured ([M+2H]/2)⁺ value is 795.30666.A structural characterization spectrogram is shown in FIG. 5.

A reaction route in the present example is as follows:

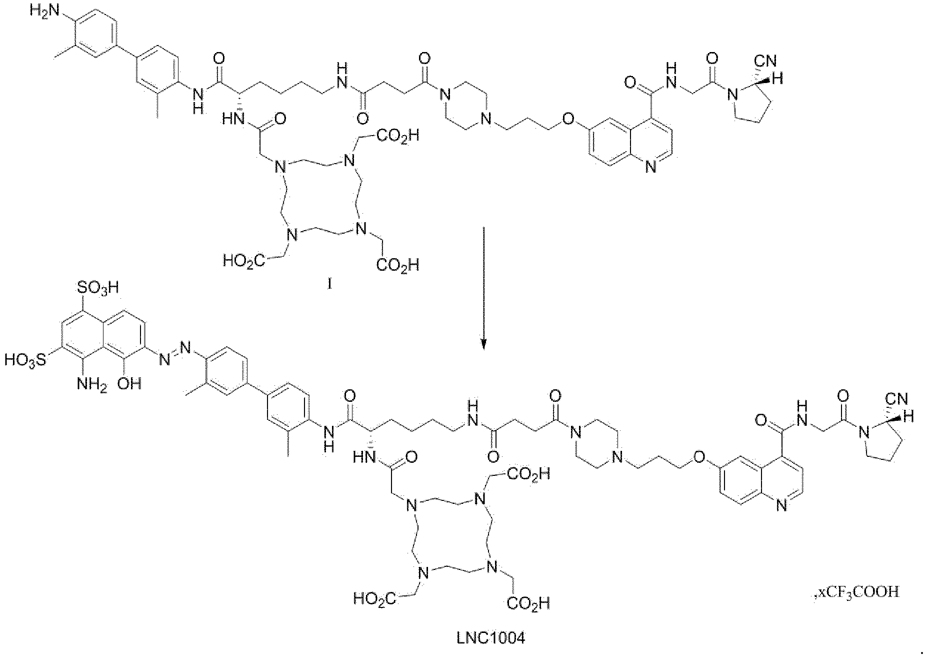

### Comparative Example 1:

### Synthesis of a compound 2

Tert-butyl (4'-amino-3,3'-dimethyl -[1,1'-biphenyl]-4-yl)carbamate (a compound 1) (0.31 g, 1.0 mmol) and 4 ml of acetonitrile were separately put into a 50 ml flask, 1.5 ml of 2 M hydrochloric acid was dropped into the reaction flask for a reaction for 15 minutes under an ice bath, and then sodium nitrite (0.068 g, 1.0 mmol) was dissolved in 2 ml of water and dropped into the reaction flask for a reaction for half an hour to obtain a solution A for later use.In addition, another 50 ml reaction flask was prepared, a 1-amino-8-naphthol-2,4-disulfonic acid monosodium salt (0.33 g, 1.0 mmol), sodium carbonate (0.105 g, 1.0 mmol), and 5 ml of water were added to constitute a solution B, and the solution A was slowly dropped into the solution B under an ice bath and stirred for a reaction for 2 h under the ice bath. Purification by preparative liquid chromatography was performed, followed by freeze-drying to obtain a pure compound 2 with a yield of 47%.

### Synthesis of a compound 3

Under ice bath conditions, the compound 2 (0.52 g, 1.0 mmol) was dissolved in trifluoroacetic acid, the system was heated to room temperature for a reaction for 2 hours, and after completion of the reaction, reduced pressure distillation was performed to remove a solvent so as to obtain a crude product. The crude product was purified by preparative liquid chromatography, and freeze-dried to obtain a pure compound 3 with a yield of 73%.

### Synthesis of a compound 4

The compound 3 (0.54 g, 1.0 mmol), Boc-Lys(Fmoc)-OH (0.47 g, 1.0 mmol), HATU (0.38 g, 1.0 mmol), N,N-diisopropylethylamine (0.26 g, 2.0 mmol), and 10 ml of N,N-dimethylformamide were separately put into a 100 ml flask. A reaction mixture was stirred until completion of a reaction, and reduced pressure distillation was performed to remove a solvent so as to obtain a crude product. The crude product was purified by preparative liquid chromatography, and freeze-dried to obtain a pure compound 4 with a yield of 47%.

### Preparation of a compound 5

The compound 4 was subjected to deprotection of Fmoc at room temperature by using piperidine (20% [v/v]). A reaction mixture was stirred for 1 hour, and then DMF was removed under high vacuum. A residue was purified by preparative liquid chromatography. A separation yield was 67%.Succinic anhydride (500 mg, 5 mmol) was dissolved in 5 ml of DMF and then added to an intermediate (0.5 mmol, 375 mg) of the compound 4 without Fmoc protection, and then 10 mmol of DIPEA was added. A mixture was stirred at room temperature for 12 hours until being completely converted into a compound 5. Then, the DMF was removed under high vacuum. A residue was purified by preparative liquid chromatography (278 mg, yield: 64%).

### Preparation of a compound 8

Tert-butyl (S)-4-(3-((4-((2-(2-cyanopyrrolidin-1-yl)-2-oxoethyl)carbamoyl)quino -lin-6-yl)oxy)propyl)piperazin-1-carboxylate (a compound 6) (110 mg, 0.2 mmol) and 4-methylbenzenesulfonic acid monohydrate (380 mg, 2 mmol) were dissolved in 10 ml of acetonitrile. Reactants were shaken overnight at 45°C to obtain a compound 7.Rotary evaporation was performed to remove the acetonitrile, and a residue was dissolved in DMF. Then, the compound 5 (174 mg, 0.2 mmol), N,N-diisopropylethylamine (129 mg, 1 mmol), and HATU (76 mg, 0.2 mmol) were added. A reaction mixture was stirred at room temperature for 6 hours to obtain a compound 8.The DMF was removed under high vacuum, and a residue was purified by preparative liquid chromatography (210 mg, yield: 81%).

### Preparation of LNC 1004

At room temperature, 10% TFA was used to remove a BOC group of the compound 8 (26 mg, 0.02 mmol) in DCM (v/v) for 1 hour. Then, after the TFA and the DCM were removed by an argon flow, a residue was enabled to undergo a reaction with 1.2 equivalent of DOTA-NHS ester and 8 equivalent of DIPEA in DMF. After stirring at room temperature for 2-3 hours, a reaction mixture was purified by preparative liquid chromatography to obtain a desired product (23 mg, yield: 73%).

A reaction route is as follows:

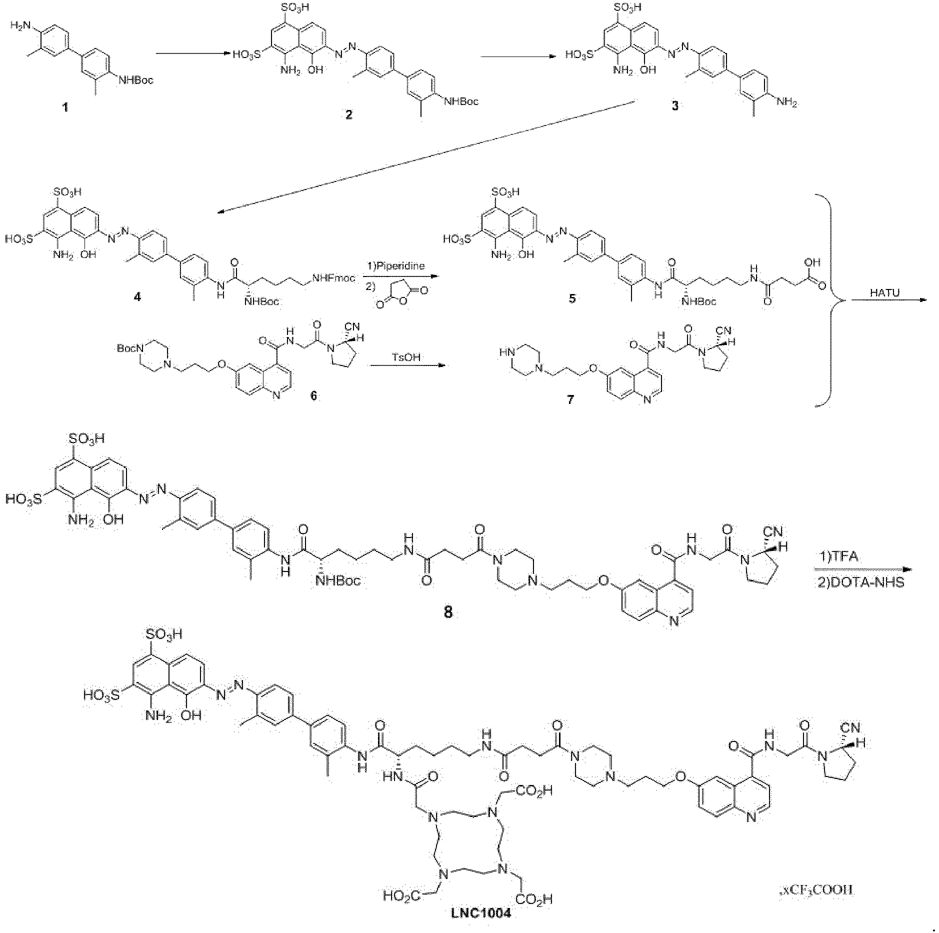

As can be seen through comparison between Examples 1-2 and Comparative Example 1 of the present invention, the LNC1004 is synthesized by reactions in 7 steps in Comparative Example 1, the post-treatment in each step needs purification by preparative liquid chromatography, and a total yield is 4.09%.Meanwhile, according to integral synthesis routes in Examples 1 and 2 of the present invention, the LNC1004 is synthesized by reactions in 6 steps, wherein only the last two steps need purification by preparative liquid chromatography, and a total yield is 20.38%.It can be seen that the synthesis method of the present invention can significantly improve the yield of LNC1004.

## Claims

1. An intermediate for synthesis of Evans blue modified FAPI, wherein the intermediate has a structure as shown in Formula (I):

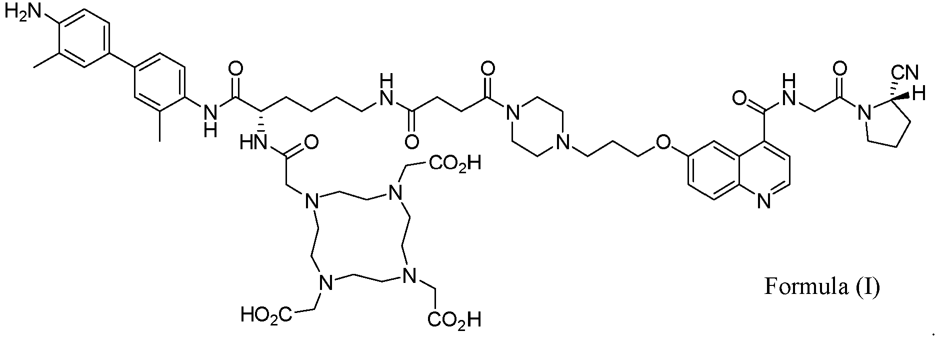

2. A method for preparing the intermediate according to claim 1, wherein the method comprises the following steps:
step a: subjecting (S)-N-(2-(2-cyanopyrrolidin-1-yl)-2-oxoethyl)-6-(3-(piperazin-1-yl) propoxy)quinolin-4-formamide (a compound II) to a reaction with succinic anhydride to obtain a compound III;
step b: subjecting the compound III to a coupling reaction with tert-butyl (S)-(6-amino-1-((4'-amino-3,3'-dimethyl-[1,1'-biphenyl]-4-yl)amino)-1-oxohexyl-2-yl)carbamate (a compound IV) and treatment to obtain a compound V;
step c: subjecting the compound V to a deprotection reaction to obtain a compound VI, and then to a substitution reaction and treatment to obtain a compound VII; and
step d: subjecting the compound VII to a hydrolysis reaction and treatment to obtain the intermediate having the structure of Formula I;
and a specific reaction formula is shown as follows:

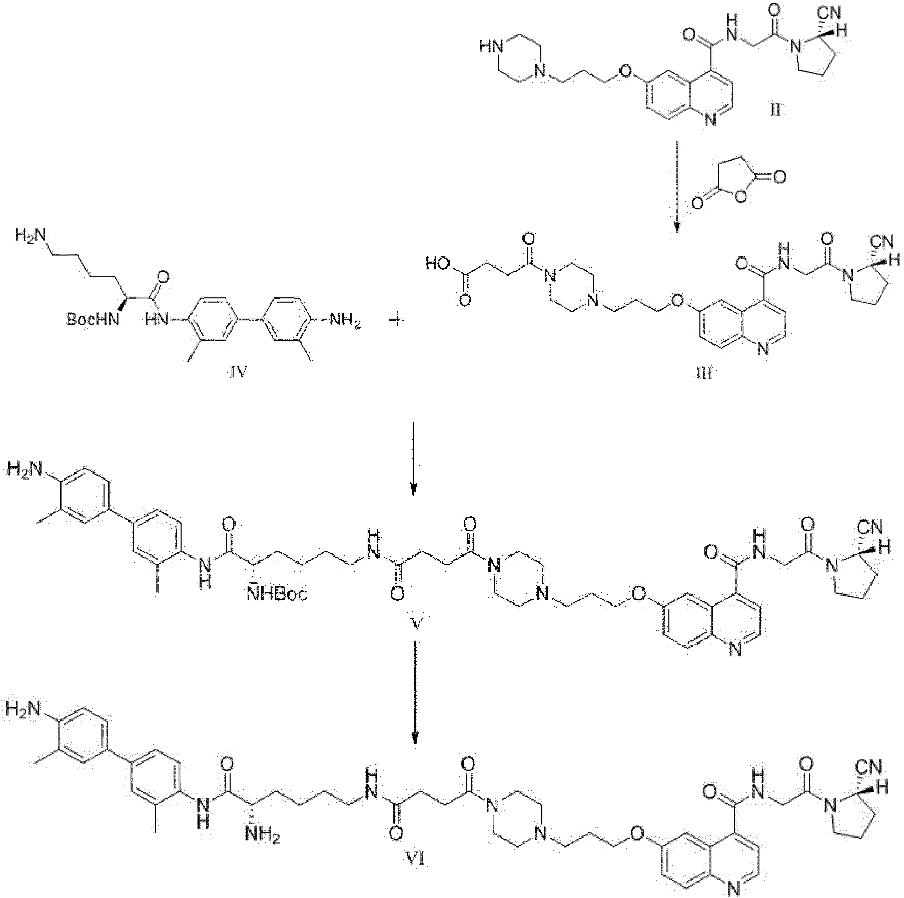

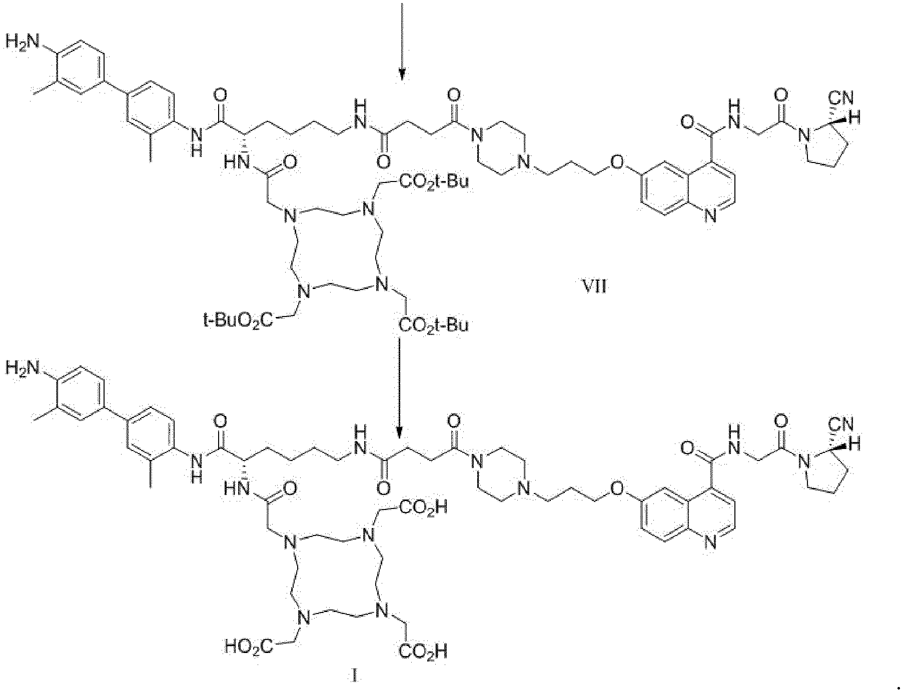

3. The method according to claim 2, wherein the step b specifically comprises: in an N,N-dimethylformamide solvent, adding the compound III obtained in the step a and the tert-butyl (S)-(6-amino-1-((4'-amino-3,3'-dimethyl-[1,1'-biphenyl]-4-yl)amino)-1-oxohexyl -2-yl)carbamate, then adding a condensing agent and an organic alkali, performing stirring for a reaction at 20-45°C for 1-8 hours, and performing treatment to obtain the compound V.

4. The method according to claim 3, wherein the condensing agent comprises any one of HATU, HBTU, TBTU, TSTU, PyAOP, and PyBOP.

5. The method according to claim 3, wherein the condensing agent is HATU.

6. The method according to claim 3, wherein the organic alkali comprises any one of N,N-diisopropylethylamine and triethylamine.

7. The method according to claim 3, wherein the organic alkali is N,N-diisopropylethylamine.

8. The method according to claim 2, wherein the step c specifically comprises: in an organic solvent, adding the compound V obtained in the step b, then adding an organic acid, performing stirring for a reaction at 20-45°C for 1-8 hours to obtain the compound VI, then adding an excess amount of an organic alkali, adding tri-tert-butyl 2,2',2"-(10-(2-((2,5-dioxopyrrolidin-1-yl)oxy)-2-oxoethyl)-1,4,7,10-tetraazacyclododecan-1,4,7-triyl)triacetate, performing stirring for a reaction at 15-45°C for 1-8 hours, and performing treatment to obtain the compound VII.

9. The method according to claim 8, wherein the organic solvent comprises any one of dichloromethane, N,N-dimethylformamide, or a mixture of the two.

10. The method according to claim 8, wherein the organic solvent is N,N-dimethylformamide.

11. The method according to claim 8, wherein the organic acid comprises any one of trifluoroacetic acid and p-toluenesulfonic acid.

12. The method according to claim 8, wherein the organic acid is trifluoroacetic acid.

13. The method according to claim 8, wherein the organic alkali comprises any one of N,N-diisopropylethylamine and triethylamine.

14. The method according to claim 8, wherein the organic alkali is N,N-diisopropylethylamine.

15. The method according to claim 2, wherein the step d specifically comprises: in an organic solvent, adding the compound VII obtained in the step c, then adding an organic acid, performing stirring for a reaction at 20-45°C for 1-8 hours, and performing treatment to obtain the intermediate having the structure of Formula I.

16. The method according to claim 15, wherein the organic acid comprises any one of trifluoroacetic acid and p-toluenesulfonic acid.

17. The method according to claim 15, wherein the organic acid is trifluoroacetic acid.

18. Use of the intermediate according to claim 1 in synthesis of Evans blue modified FAPI, wherein the use comprises the following steps: dissolving the intermediate having the structure of Formula I in water, sequentially adding a hydrochloric acid solution and sodium nitrite for a reaction to generate a diazonium salt solution under stirring conditions at -5°C to 25°C, then mixing a 1-amino-8-naphthol-2,4-disulfonic acid monosodium salt and sodium bicarbonate for an acid-base neutralization reaction to generate an aqueous solution of the 1-amino-8-naphthol-2,4-disulfonic acid monosodium salt and the sodium bicarbonate, dropping the diazonium salt solution into the aqueous solution of the 1-amino-8-naphthol-2,4-disulfonic acid monosodium salt and the sodium bicarbonate for a reaction for 1-8 hours under stirring at -5°C to 25°C, and performing post-treatment to obtain Evans blue modified FAPI; and a specific reaction formula is shown as follows:

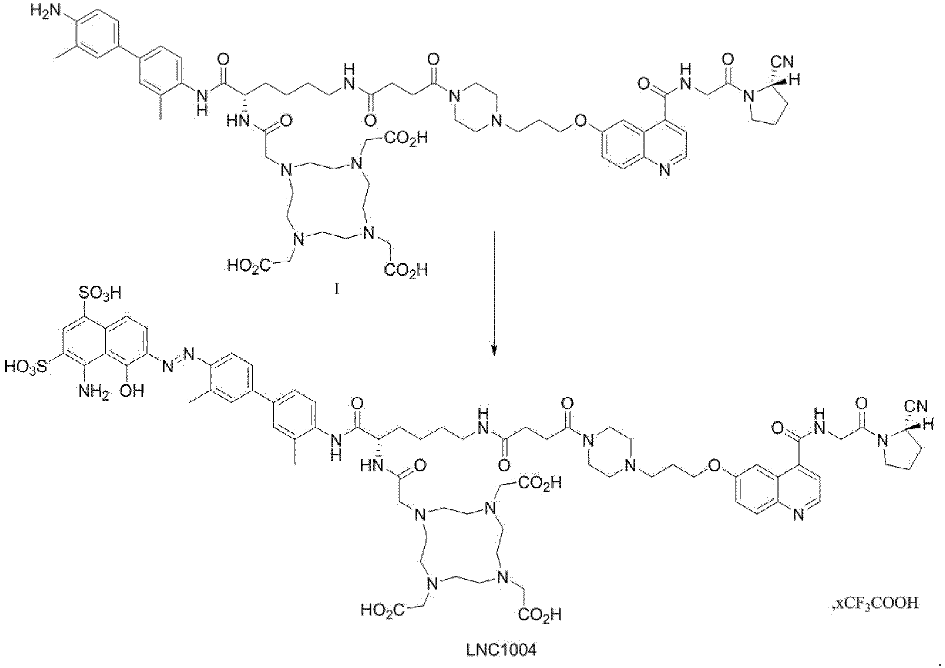

19. The use according to claim 18, wherein a molar concentration of the hydrochloric acid solution is 0.5-6 mol/L.

20. The use according to claim 18, wherein a molar concentration of the hydrochloric acid solution is 1-3 mol/L.

21. The use according to claim 18, wherein a molar ratio of hydrochloric acid to the intermediate after addition of the hydrochloric acid solution is 1.0-3.0.

22. The use according to claim 18, wherein a molar ratio of hydrochloric acid to the intermediate after addition of the hydrochloric acid solution is 1.5-2.0.

23. The use according to claim 18, wherein a molar ratio of the sodium nitrite to the intermediate is 1.0-3.0.

24. The use according to claim 18, wherein a molar ratio of the sodium nitrite to the intermediate is 1.0-1.5.

25. The use according to claim 18, wherein a molar ratio of the 1-amino-8-naphthol-2,4-disulfonic acid monosodium salt to the intermediate is 1.0-3.0.

26. The use according to claim 18, wherein a molar ratio of the 1-amino-8-naphthol-2,4-disulfonic acid monosodium salt to the intermediate is 1.0-1.5.

27. The use according to claim 18, wherein a molar ratio of the sodium bicarbonate to the intermediate is 5.0-10.0.

28. The use according to claim 18, wherein a molar ratio of the sodium bicarbonate to the intermediate is 8.0-10.0.
